# EUROPEAN PATENT APPLICATION

(11) **EP 3 324 319 A1**
(43) Date of publication of application: **23.05.2018**
(21) Application number: 16203005.0
(22) Date of filing: 08.12.2016
(51) Int. Cl.: G06F 19/00

(54) **METHOD OF MAPPING A MEDICAL IMAGING ACQUISITION PROTOCOL TO A LEXICON**

(30) Priority: 22.11.2016 EP 16200055
(71) Applicant: Siemens Healthcare GmbH, 91052 Erlangen (DE)
(72) Inventor: Guendel, Sebastian, 91056 Erlangen (DE); Kramer, Martin, 91058 Erlangen (DE); Pauly, Olivier, 81241 München (DE)

(57) **Abstract**

The invention describes a method of mapping an acquisition protocol (P) to an acquisition protocol lexicon (X), which method comprises the steps of extracting a plurality of tags (100) from the acquisition protocol (P); performing text preprocessing on the extracted tags (100); converting the preprocessed text (110) into an input feature set (120) for a classifier (13); and applying the classifier (13) to associate the input feature set (120) with one or more entries (ID) of the acquisition protocol lexicon (X).The invention further describes a protocol mapper (1) realised to map an acquisition protocol (P) to entries (ID) of an acquisition protocol lexicon (X); and a computer program product for carrying out the steps of the method.

## Description

The invention describes a method of mapping an acquisition protocol to an acquisition protocol lexicon; and a protocol mapper.

The collection of settings and parameters defining a medical imaging examination is called the "exam protocol" or "acquisition protocol". An acquisition protocol defines the actions to be performed on a patient, such as the scan modality, whether or not a contrast agent is to be used, whether a surgical instrument will be used, the number of views to be acquired, the relevant population such as paediatric, trimester, etc. Each acquisition protocol may be given a unique identifier or protocol ID. The different procedures that are performed in an institution such as a hospital or a radiological practice are usually defined internally in that institution. To setup an image acquisition procedure, it may be sufficient for the clinician or medical technical assistant to enter the protocol ID into a workstation or scanning apparatus. The acquisition protocol and/or the protocol ID, as wells as other information related to the institution and the patient, can be saved along with the image data. To facilitate the exchange, comparison and interpretation of imaging results between medical personnel and institutions, the additional data is often generated and stored using the standard DICOM (Digital Imaging and Communications in Medicine) format. This standard was specifically developed to handle data related to all stages of medical imaging (image acquisition, storage, transmission, exchange, etc.), and is widely used by institutions such as hospitals, surgical practices, medical imaging service providers, etc.

The same imaging procedure may be given different names by different institutions. For instance, one institution may define an abdomen/pelvis CT exam without contrast agent as "ABD/PEL WO" while another institute may use "CT Abdomen Pelvis without Contrast" to define the same exam or imaging procedure. However, the exam quality and radiation dose depends to a great extent on the acquisition protocol that was used to set up the imaging procedure. Furthermore, it is very important to be able to understand, reproduce, and compare acquisition protocols used by different institutions. This would make it necessary for all institutions to adopt a unifying protocol. An example of such a unifying protocol is given in the radiological lexicon named RadLex^{®} (often referred to as the "RadLex^{®} Playbook" or simply the "Playbook"), which has been compiled with the aim of providing a unified description for all possible kinds of imaging acquisition procedure, and associating each procedure with a unique identifier, its RPID (Radlex protocol identifier). While this unifying lexicon is not an official standard, many institutions recognise the need to convert past (and future) acquisition protocols to a common lexicon such as that provided by RadLex^{®}. However, not all operators of the various kinds of medical imaging acquisition devices are sufficiently familiar with the protocols of such a unifying lexicon. Furthermore, it is not always possible for an operator to simply "translate" the protocol of that institution into a protocol of the unifying lexicon.

In one approach to solving this problem, a software program or tool applies a set of "hand-crafted" predicates or rules to extract the relevant information from an acquisition protocol, re-formats the information in keeping with the unifying protocol of a lexicon such as the RadLex^{®} Playbook, and maps the reformatted protocol to the lexicon in order to find the RPID that matches the acquisition protocol. However, a limitation of this approach is that it is necessary to compile a comprehensive rule set in the first place, and then to manually maintain and update this rule set. Furthermore, this approach requires a comprehensive medical ontology database as well as a search engine in order to correctly map a freely composed acquisition protocol to a corresponding lexicon protocol. A further drawback is that each time another institution or another exam protocol is added, the rule set needs to be manually updated to augment it with the new information, and the updated rule set must be provided to all users of the tool.

It is therefore an object of the invention to provide an improved way of assisting institutions in their endeavour to apply the acquisition protocols defined in a unifying lexicon.

This object is achieved by the method of claim 1 of mapping an acquisition protocol to an acquisition protocol lexicon; and by the protocol mapper of claim 7.

According to the invention, the method of mapping an acquisition protocol to an acquisition protocol lexicon comprises the steps of extracting a plurality of tags from the acquisition protocol; performing text pre-processing on the extracted tags; converting the pre-processed text into an input feature set for a classifier; and applying the classifier to associate the input feature set with one or more entries of the acquisition protocol lexicon.

In the context of the invention, the expression "mapping an acquisition protocol to an acquisition protocol lexicon" is to be understood as identifying one or more lexicon entries that are the most likely equivalents of the input acquisition protocol. It may be assumed that the acquisition protocol is a medical imaging acquisition protocol for an intended imaging procedure, and that the acquisition protocol is informal, i.e. it is put together or composed by the user (the operator of the imaging device, usually a clinician or medical technical assistant, for example) without strict adherence to any "global" formulation constraints, since such constraints do not exist at present. The user's input may at best adhere to local formulation guidelines of that institution, but such formulation guidelines will generally differ widely between institutions, as explained above. Therefore, the acquisition protocol put together by a user may be considered to be "informal" or "freely composed", in the sense that users at different institutions may arrive at significantly different acquisition protocols for the same intended procedure.

The inventive method can be used to associate any acquisition protocol with one or more entries in the lexicon. An advantage of the mapping method according to the invention is that it is an approach based on a machine learning pipeline. Therefore, there is no need to manually create and maintain a rule set, since any rules are learned directly from the input data fed to the classifier. This means that advantageous savings can be made in time and costs. Furthermore, when a new institution or a new protocol is added, the inventive method can easily adapt by automatically accumulating the new information and learning from it.

According to the invention, the protocol mapper is realised to map an acquisition protocol to an acquisition protocol lexicon and comprises a tag extraction module realized to extract a plurality of tags from an acquisition protocol; a pre-processing module realized to perform text pre-processing on the extracted tags; a feature extraction module realized to convert the pre-processed text into an input feature set; and a classifier realized to associate an input feature set with one or more entries of the acquisition protocol lexicon.

An advantage of the protocol mapper according to the invention is that relatively little effort need be expended in order to achieve a reliable and accurate tool which can provide a user with a list of relevant protocol descriptions that best match the intended imaging procedure. This assists the user in making an accurate selection from the list of lexicon entries returned by the classifier. In this way, a user at any institution can apply the guidelines of that instruction to assemble an "informal" acquisition protocol, and can quickly receive a list of entries from the more "formal" lexicon, which best match that acquisition protocol. The user can then choose the most suitable entry from the list, and use this to program the device for the planned imaging procedure.

Particularly advantageous embodiments and features of the invention are given by the dependent claims, as revealed in the following description. Features of different claim categories may be combined as appropriate to give further embodiments not described herein.

In the following, it may be assumed that the unifying lexicon is the RadLEx^{®} playbook, which is already widely used as a kind of standard for defining imaging procedures such as Ultrasound, X-ray, CT, MRI, fluoroscopy, etc. A specific imaging procedure is defined in the RadLEx^{®} playbook by a specific identifier, called its "RPID". For example, a procedure for performing an ultrasound of the liver has the identifier RPID5928 in the RadLEx^{®} playbook, with the associated description "US Abdomen Limited Liver".

The terms "protocol mapper", "classification pipeline" and "machine learning pipeline" may be regarded as synonyms in the context of the invention, and these terms may therefore be used interchangeably in the following.

The local protocol for acquiring image data at a certain institution differ significantly from an equivalent protocol of a unifying lexicon. As explained above, it is necessary to identify that imaging procedure using its specific RPID if the results of the imaging procedure are to be viewed at a different institution, for example. The inventive method provides a reliable and quick way of obtaining the most likely RPID for an informally composed local protocol.

The tag extraction module is preferably realized to identify tags that correspond to parameters defined in the DICOM standard. Preferably, the extracted tags comprise at least a "body region" tag, a "local protocol name" tag, an "institution" tag and a "modality" tag. The "modality" tag defines the imaging modality, for example CT (computed tomography), FL (fluoroscopy), US (ultrasound), etc. The "institution" tag is a unique identifier, so that each institution can be defined by a unique number or customer number. For example, allocation of the institution tag can be done by a counter that is incremented for each new institution that becomes a customer of the inventive mapping service. The "body region" tag defines the part of the body to be imaged, for example "chest", "head" etc. The "local protocol name" tag is the text used by an institution to define a certain imaging procedure.

The extracted body region tag and protocol name tag are subject to lexical thinning in the text pre-processing step, for example to remove non-alphanumeric "special" characters, to discard any one-character or two-character terms, to convert all letters to lower-case, etc. After this step of lexical thinning, the feature extraction module converts the remaining text into an input feature set. This will comprise an entry for the modality (e.g. "CT"), an entry for the institution (e.g. "4"), and lexically thinned entries for the body region and protocol name. In a preferred embodiment of the invention, in addition to the modality and institution entries, the input feature vector comprises a sparse signature compiled using a bag-of-words technique. In the bag-of-words technique, an algorithm goes over all words in a training set of local protocol names and body regions to create a dictionary or "bag of words". Using this dictionary, it is then possible to describe the "body region" word(s) or "protocol name" word(s) by the number of times those word appear in the dictionary or "bag of words". The contribution of each word or term in the dictionary can be weighted according to term frequency (TF), inverse document frequency (IDF) or a combination of both. A sparse signature for the body region and/or protocol name can then be created with this information.

This feature vector or feature set is then fed to the classifier or "predictive model", which applies a suitable classification algorithm to associate or map that feature set to one or more entries of the lexicon. In a particularly preferred embodiment of the invention, the classifier applies a random forest algorithm to associate the input feature vector with one or more entries of the acquisition protocol lexicon. Alternatively, the classifier might use a support vector machine (SVM) or a neural network to associate an input feature vector with one or more entries of the acquisition protocol lexicon. Preferably, the inventive method comprises an initial step of training the classifier using any appropriate machine learning algorithm that is able to learn the parameters of the classifier's predictive model using a suitable dataset or input.

The inventive protocol mapper is suited for implementation in the cloud, i.e. it can be realised in a cloud computing platform. Certain modules of the protocol mapper such as the tag extraction module can be implemented in a web-based application. This can interface with a user via an internet browser, for example, so that the user can enter information and view results in such a browser window. Other modules of the protocol mapper such as the pre-processing module, the feature extraction module and the classifier can be implemented in a web-based service. The web-based service can be realised to communicate with multiple web applications and/or multiple instances of the same web application. To this end, the tag extraction module of a web application is preferably realized to convert upload data for the web-based service input into a suitable format such as JSON (JavaScript object notation). Similarly, the web application is preferably realized to convert the classifier results from such a format in order to present the mapping results to the user, for example as a table of entries and their probabilities. A web-based application for the inventive protocol mapper can be adapted to receive acquisition protocols originating from a single institution such as a hospital or a radiology practice. In a particularly preferred embodiment of the invention, a web-based application for the inventive protocol mapper is adapted to receive acquisition protocols from a plurality of institutions and/or for a plurality of modalities. Equally, a web-based application for the inventive protocol mapper can be adapted to receive acquisition protocols relating to a specific modality, for example only protocols relating to computed tomography. In a further preferred embodiment of the invention, it is possible to use "modality" and "institution" a *priori* to build a modality-specific and/or institution-specific mapping pipeline, or a *posteriori* to refine the prediction results (for example by filtering out classes that do not contain the modality of interest). An institution-specific model could be initialized with a generic model and then refined by integrating user feedback in an online learning procedure.

The steps of the inventive method can be implemented as a computer program product when this is loaded into a memory of a programmable device. For example, any method steps relating to user dialog can be implemented as a computer program product running on a server hosting the web application, while the remaining method steps can be implemented as a computer program product running on a server hosting the protocol mapping web service.

Other objects and features of the present invention will become apparent from the following detailed descriptions considered in conjunction with the accompanying drawings. It is to be understood, however, that the drawings are designed solely for the purposes of illustration and not as a definition of the limits of the invention.
- Fig. 1: shows an embodiment of the inventive protocol mapper during a protocol mapping procedure;
- Fig. 2: illustrates a dialog between a user and the inventive protocol mapper;
- Fig. 3: shows steps of the inventive method;
- Fig. 4: shows a table of words resulting from a bag-of-words algorithm;
- Fig. 5: shows an embodiment of the inventive protocol mapper during a training procedure;
- Fig. 6: shows training data used to train or retrain the classifier of the inventive protocol mapper;
- Fig. 7: shows a flow chart illustrating the steps in a prior art method of obtaining a protocol from a unifying lexicon.

In the diagrams, like numbers refer to like objects throughout. Objects in the diagrams are not necessarily drawn to scale.

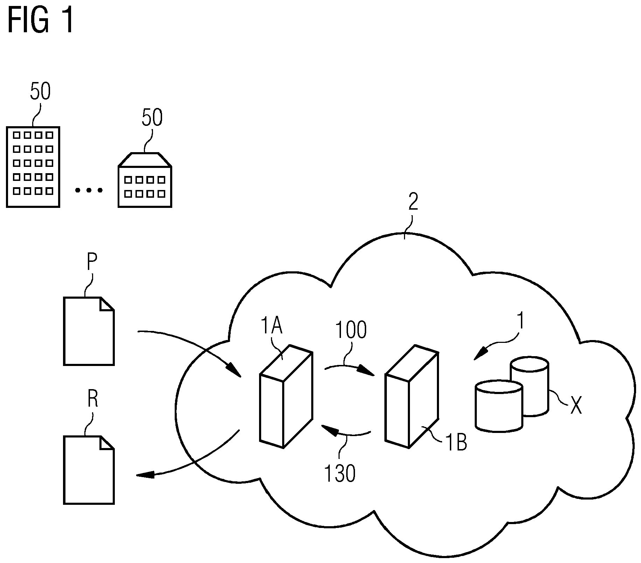

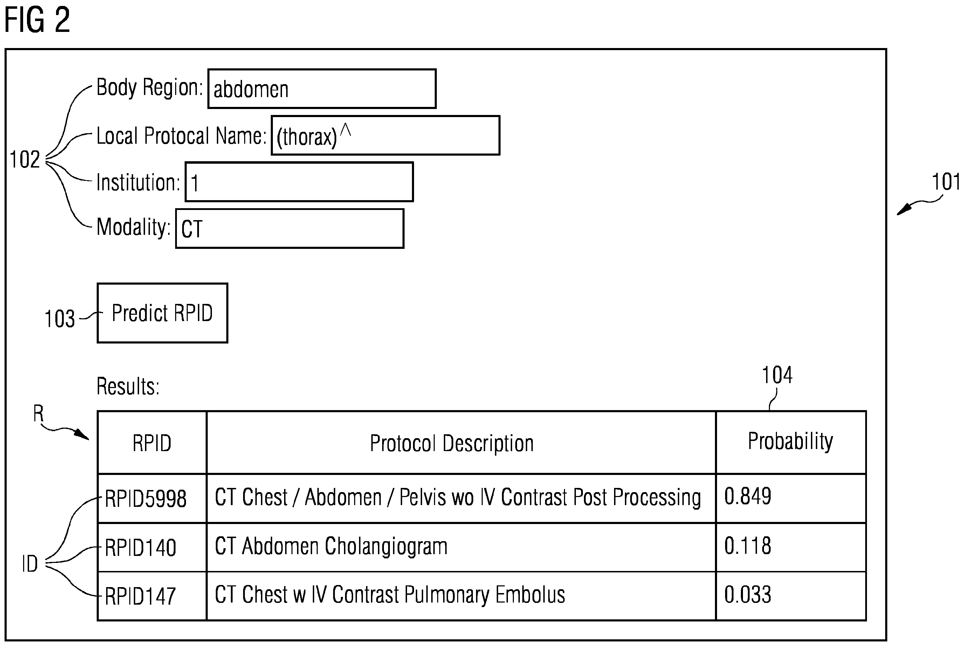

Fig. 1 shows an embodiment of the inventive protocol mapper 1 implemented in a cloud computing environment 2. Using some appropriate interface such as a browser window of a web application 1A (represented by a server symbol in the cloud 2), a user at an institution 50 can enter an acquisition protocol P, for example by entering data into fields named according to parameters used in the DICOM standard. Fig. 2 shows an example of protocol fields 102 of an acquisition protocol for completion by the user in a browser window 101 of such a web application 1A. The web application 1A converts the acquisition protocol P into a suitable format such as JSON and transmits this upload data 100 to a protocol mapping web service 1B (also represented by a server symbol in the cloud 2). This in turn performs the feature extraction and classification steps of the inventive method to identify the entry or entries in a protocol lexicon X that would be the most likely candidates to match the acquisition protocol P. The web service 1B returns a list of probabilities as download data 130 (also in JSON format) to the web application 1A, which converts the download data 130 into a format which can be viewed by the user, for example in the same browser window 101 of the web application 1A. In the exemplary illustration of Fig. 2, the user is presented with a table 104 in the browser window 101, showing a list of candidate RPIDs ID and their associated probabilities. Fig. 2 also indicates some DICOM parameter input fields in the browser window 101. Here, the user has entered information for the "Body Region", "Local Protocol Name", "Institution" and "Modality" DICOM parameters. After entering all relevant fields, the user has mouse-clicked the "Predict RPID" button 103, and results R of the protocol mapping are then shown in a results table 104 as a list of RPIDs ID (left-hand column), the associated protocol descriptions, and the associated probabilities (right-hand column). In this example, the information entered by the user is most likely associated with RPID5998, since the probability is 84.9%.

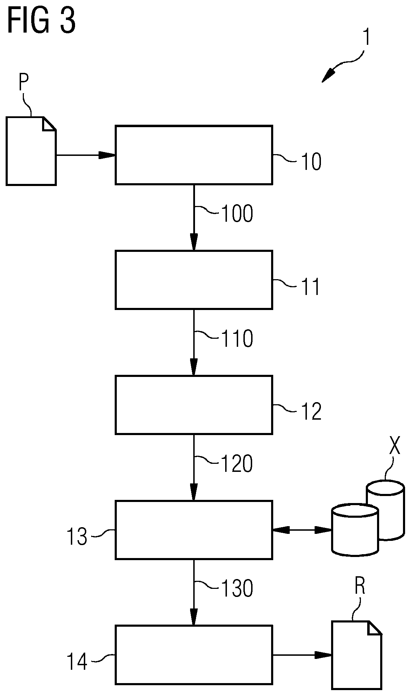

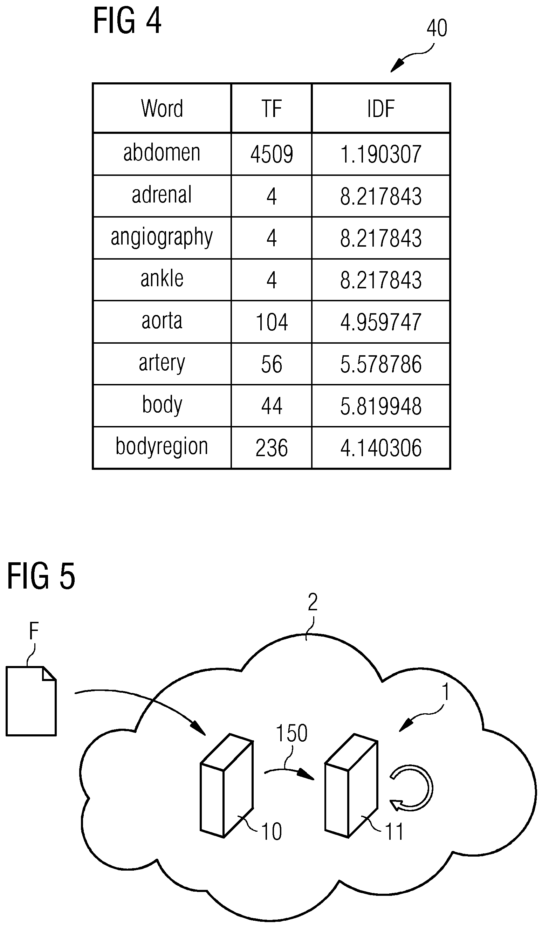

Fig. 3 shows steps in the inventive method of mapping any acquisition protocol with one or more entries of a protocol lexicon. These steps are carried out by the protocol mapper 1 as explained in Fig. 1 above. In a first stage, a tag extraction module 10 is fed with an acquisition protocol P. The tag extraction module 10 extracts a plurality of tags from the acquisition protocol P, converts these to JSON format, and forwards this upload data 100 to a pre-processing module 11, which proceeds to perform text pre-processing on the extracted tags 100. For example, the text pre-processing steps may involve converting all text to upper case or to lower case, removing all terms that comprise less than three characters, discarding all special characters, etc. In a subsequent stage, a feature extraction module 12 uses the pre-processor output 110 to assemble an input feature set 120 for a classifier 13. The input feature set 120 can be assembled or compiled in various ways. In a preferred approach, the feature set 120 includes a sparse signature which is put together from information obtained using the "bag-of-words" technique. The words associated with the "body region" and "local protocol name" tags (after the pre-processing step) are now combined with their term frequency and/or inverse document frequency in a previously prepared dictionary or "bag-of-words" for local protocol names and body regions. Fig. 4 shows part of an exemplary table 40 showing words of a dictionary in the left-hand column, the term frequency (TF) of each word in the middle column, and the inverse document frequency (IDF) of that word in the right-hand column. Returning to Fig. 3, the feature set 120 - a list of entries including body region, protocol name, institution and modality, augmented by the sparse signature values for term frequency and inverse document frequency in each case - is passed to the classifier 13, which maps the feature set to one or more entries of an acquisition protocol lexicon X such as the RadLex^{®} playbook. The classifier 13 can be realized as a random forest classifier 13, and determines which entries of the lexicon X are most likely to be associated with the input feature set 120. In the course of the work leading to the invention, the performances of different types of classifier were compared using a training dataset with several thousand entries, and the random forest classifier was found to be superior regarding the accuracy of classification (for a very large dataset comprising millions of entries, a neural network may show superior performance). The classifier 13 then returns a result 130 comprising a list of entries with associated probabilities. Since these steps are being carried out in a web application, the results 130 are encoded in suitable format such as JSON. An output formatter 14 then converts the classifier results 130 into a format that can be viewed by the user (e.g. the table 104 of RPIDs and associated probabilities as shown in Fig. 2) so that the user can select the appropriate protocol.

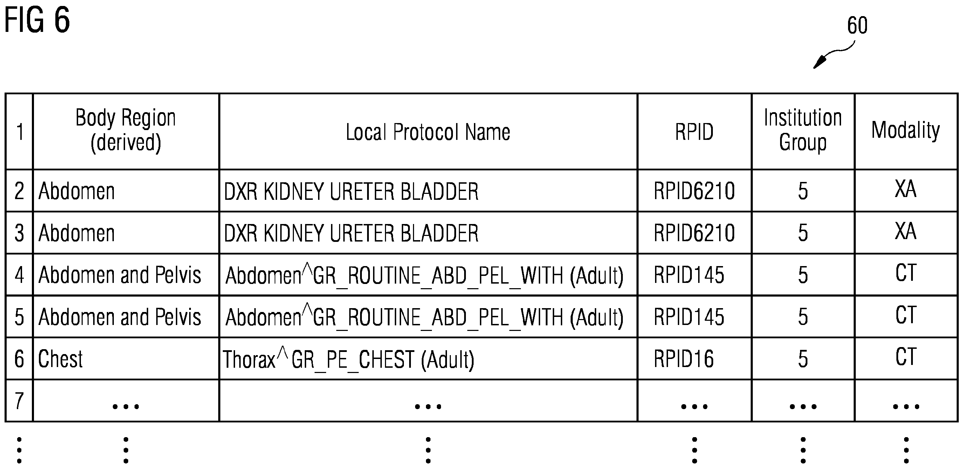

The advantage of the inventive method, as explained above, is that there is no need to create and maintain a rule set. Instead, rules are learned directly from input data that is used to train the classifier 13. Fig. 6 shows part of an exemplary table 60 of training data from an institution, which can be fed to the protocol mapper 1 in order to train the classifier 13, or to re-train the classifier 13. The table associates one or more "body region" words with a "local protocol name", the relevant lexicon ID, the number of that institution, and the imaging modality (reading each row from left to right).

The accuracy of the method can be refined continually by learning from user feedback. For example, the user may be given the opportunity to inform the web application 1B whether or not a result was correct. This feedback can be used as shown in Fig. 5 to re-train the protocol mapping web service 1B. Here, the user provides feedback F (for example by entering it into a feedback dialog window of the web application 1A), and the web interface sends feedback data 150 to the protocol mapping web service 1B. The information is used to re-train the classifier 13 of the protocol mapping web service 1B. Since re-training with new information is a straightforward procedure in the inventive protocol mapper, the addition of a new institution 50 is not in any way problematic - for example the protocol names used by that added institution can easily be incorporated to update the dictionary or bag-of-words used in the feature extraction module 10 and to retrain the classifier 13 using feedback from users at the newly added institution 50.

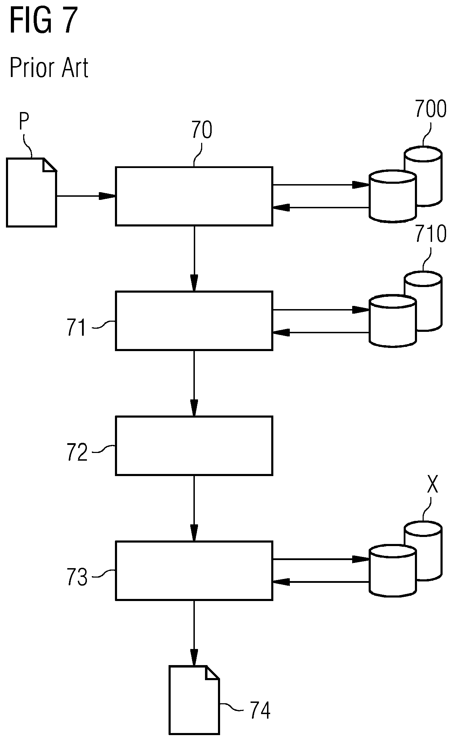

Fig. 7 shows a flow chart illustrating the steps in a prior art method of obtaining a protocol from a unifying lexicon (such as a RadLEx^{®} protocol) from an acquisition protocol P that has been formulated by a user. An exemplary acquisition protocol P may be "CT abd pelv". This acquisition protocol P is entered into a predicate extraction module 70 in a first step, so that specific predicates can be extracted from the words and terms used in the protocol. Pertinent predicates may be the modality ("CT"), the body region ("abd"), and the anatomic focus ("pelv"), for example. The predicate extraction module 70 applies a set of rules 700 to process the input text. One rule may be applied to remove special characters from the input terms, another rule may map an abbreviation to a whole word or vice versa, another rule may map a specific anatomical term to a more general term ("lung" to "chest", for example), etc. A subsequent adjustment module 71 is connected to a medical ontology database 720, so that the correct medical terminology can be extracted from the predicates. In this example, the predicate "abd" is adjusted or translated to the medical term "abdomen". In a next stage, a reformatting module 72 reformats the protocol using the adjusted predicates. The exemplary protocol becomes "CT ABDOMEN PELVIS", and is forwarded to a protocol mapper 73, which uses a search engine to search a database such as the RadLEx^{®} playbook X to identify any appropriate protocol. In the present example, the search result 74 may return "RPID839 - CT Abdomen", for example. While the prior art method can yield satisfactory results, a major drawback with this approach is that it is based on a hand-crafted set of rules 700 used in the predicate extraction module 70, and this rule set 700 must be maintained and edited manually. The success and reliability of the prior art method relies on diligent and thorough maintenance of the rule set 700. Furthermore, the rule set 700 must be updated each time a new institution is added, or every time a new acquisition protocol is added, since there are generally very significant differences in the way that personnel of different institutions have learned to formulate acquisition protocols.

Although the present invention has been disclosed in the form of preferred embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

## Claims

1. A method of mapping an acquisition protocol (P) to an acquisition protocol lexicon (X), which method comprises the steps of
- extracting a plurality of tags (100) from the acquisition protocol (P);
- performing text pre-processing on the extracted tags (100) ;
- converting the pre-processed text (110) into an input feature set (120) for a classifier (13); and
- applying the classifier (13) to associate the input feature set (120) with one or more entries (ID) of the acquisition protocol lexicon (X).

2. A method according to claim 1, wherein the extracted tags (100) comprise at least a "body region" tag, a "local protocol name" tag, an "institution" tag and a "modality" tag.

3. A method according to claim 1 or claim 2, wherein the extracted tags (100) are subject to lexical thinning in the text pre-processing step.

4. A method according to any of the preceding claims, wherein the input feature set (120) comprises a sparse signature assembled on the basis of a bag-of-words model and the results (110) of the text pre-processing step.

5. A method according to any of the preceding claims, wherein the classifier (13) applies a machine-learning algorithm, preferably a random forest algorithm, to associate an input feature set (120) with one or more entries (ID) of the acquisition protocol lexicon (X).

6. A method according to any of the preceding claims, comprising a step of re-training the classifier (13) with new information.

7. A protocol mapper (1) realised to map an acquisition protocol (P) to entries (ID) of an acquisition protocol lexicon (X), which protocol mapper () comprises
- a tag extraction module (10) realized to extract a plurality of tags (100) from an acquisition protocol (P);
- a pre-processing module (11) realized to perform text pre-processing on the extracted tags (100);
- a feature extraction module (12) realized to convert the pre-processed text (110) into an input feature set (120); and
- a classifier (13) realized to associate an input feature set (120) with one or more entries (ID) of the acquisition protocol lexicon (X).

8. A protocol mapper according to claim 7, wherein the tag extraction module (10) is realized to extract DICOM tags (100) from the acquisition protocol (P).

9. A protocol mapper according to claim 7 or claim 8, wherein the tag extraction module (10) is realized to encode the extracted tags (100) in JSON format, and wherein the output formatting module (14) is realized to decode the classifier results from JSON format.

10. A protocol mapper according to any of claims 7 to 9, adapted to receive acquisition protocols (P) from a plurality of institutions (50) and/or for a plurality of modalities.

11. A protocol mapper according to any of claims 7 to 10, adapted to receive acquisition protocols (P) originating from a single institution (50) and/or to receive acquisition protocols (P) relating to a specific modality.

12. A protocol mapper according to any of claims 7 to 11, realised in a cloud computing platform (2).

13. A computer program product for carrying out the steps of the method according to any of claims 1 to 6 when the computer program product is loaded into a memory of a programmable device.

14. A computer-readable medium on which is stored program elements that can be read and executed by a computer unit in order to perform steps of the method according to any of claims 1 to 6 when the program elements are executed by the computer unit.

## Amended claims

### Amended claims in accordance with Rule 137(2) EPC.

1. A method of mapping an acquisition protocol (P) to an acquisition protocol lexicon (X), which method comprises the steps of
- extracting a plurality of tags (100) from the acquisition protocol (P) of a medical imaging examination;
- performing text pre-processing on the extracted tags (100) ;
- converting the pre-processed text (110) into an input feature set (120) for a classifier (13); and
- applying the classifier (13) to associate the input feature set (120) with one or more entries (ID) of the acquisition protocol lexicon (X).

2. A method according to claim 1, wherein the extracted tags (100) comprise at least a "body region" tag, a "local protocol name" tag, an "institution" tag and a "modality" tag.

3. A method according to claim 1 or claim 2, wherein the extracted tags (100) are subject to lexical thinning in the text pre-processing step.

4. A method according to any of the preceding claims, wherein the input feature set (120) comprises a sparse signature assembled on the basis of a bag-of-words model and the results (110) of the text pre-processing step.

5. A method according to any of the preceding claims, wherein the classifier (13) applies a machine-learning algorithm, preferably a random forest algorithm, to associate an input feature set (120) with one or more entries (ID) of the acquisition protocol lexicon (X).

6. A method according to any of the preceding claims, comprising a step of re-training the classifier (13) with new information.

7. A protocol mapper (1) realised to map an acquisition protocol (P) to entries (ID) of an acquisition protocol lexicon (X), which protocol mapper (1) comprises
- a tag extraction module (10) realized to extract a plurality of tags (100) from an acquisition protocol (P);
- a pre-processing module (11) realized to perform text pre-processing on the extracted tags (100);
- a feature extraction module (12) realized to convert the pre-processed text (110) into an input feature set (120); and
- a classifier (13) realized to associate an input feature set (120) with one or more entries (ID) of the acquisition protocol lexicon (X).

8. A protocol mapper according to claim 7, wherein the tag extraction module (10) is realized to extract DICOM tags (100) from the acquisition protocol (P).

9. A protocol mapper according to claim 7 or claim 8, wherein the tag extraction module (10) is realized to encode the extracted tags (100) in JSON format, and wherein the protocol mapper (1) further comprises an output formatting module (14) realized to decode the classifier results from JSON format.

10. A protocol mapper according to any of claims 7 to 9, adapted to receive acquisition protocols (P) from a plurality of institutions (50) and/or for a plurality of modalities.

11. A protocol mapper according to any of claims 7 to 10, adapted to receive acquisition protocols (P) originating from a single institution (50) and/or to receive acquisition protocols (P) relating to a specific modality.

12. A protocol mapper according to any of claims 7 to 11, realised in a cloud computing platform (2).

13. A computer program product for carrying out the steps of the method according to any of claims 1 to 6 when the computer program product is loaded into a memory of a programmable device.

14. A computer-readable medium on which is stored program elements that can be read and executed by a computer unit in order to perform steps of the method according to any of claims 1 to 6 when the program elements are executed by the computer unit.
